# EUROPEAN PATENT APPLICATION

(11) **EP 4 847 016 A1**
(43) Date of publication of application: **02.12.2026**
(21) Application number: 25745140.1
(22) Date of filing: 22.01.2025
(51) Int. Cl.: A61K 31/135, A61K 9/06, A61K 9/08, A61P 25/02, A61P 27/02

(54) **PHARMACEUTICAL COMPOSITION, BLEPHAROSPASM TREATMENT AGENT, HYPERSENSITIVITY OR DYSTONIA TREATMENT AGENT, AND SYMPTOM MODEL MOUSE**

(30) Priority: 24.01.2024 JP 2024008809
(71) Applicant: National University Corporation Hokkaido University, Hokkaido 060-0808 (JP)
(72) Inventor: TAGAWA Yoshiaki, Sapporo-shi, Hokkaido 060-0808 (JP)
(74) Representative: Cabinet Becker et Associés
(86) International application number: PCT/JP2025/001837
(87) International publication number: WO 2025/159099

(57) **Abstract**

A pharmaceutical composition, a blepharospasm treatment agent, and a hypersensitivity or dystonia treatment agent, which have a high effect as treatment such as therapy for blepharospasm and the like, which have conventionally been difficult to treat or for which effective treatment has not been performed, and impose a small burden on patients, and a symptom model mouse for symptoms such as blepharospasm, hypersensitivity, or dystonia are provided. The pharmaceutical composition is a pharmaceutical composition containing tramadol or a salt thereof as an active ingredient, for administration to and treatment of a subject exhibiting symptoms of blepharospasm or ocular hypersensitivity. In addition, the pharmaceutical composition contains 0.01 to 2.0% w/v of the tramadol or a salt thereof. In addition, the present invention is a medicament including those, a blepharospasm treatment agent, and a hypersensitivity or dystonia treatment agent. In addition, the present invention is a symptom model mouse for symptoms such as blepharospasm, hypersensitivity, or dystonia.

## Description

### TECHNICAL FIELD

The present invention relates to a pharmaceutical composition for use in treatment of blepharospasm and the like, that is, therapy, prevention, and the like of blepharospasm and ocular hypersensitivity, an ophthalmic solution and the like using the pharmaceutical composition, a treatment agent, and a symptom model mouse for use in verification of blepharospasm and ocular hypersensitivity.

The present application claims priority based on Japanese Patent Application No. 2024-008809 filed on January 24, 2024, the contents of which are incorporated herein by reference.

### BACKGROUND ART

Blepharospasm is a disease exhibiting symptoms such as "difficulty opening the eyes", "feeling more comfortable with the eyes closed", "light is dazzling", and "eye pain", and is defined as unintended involuntary movement of the eyelid (dystonia; symptoms such as the eyes not opening despite wanting to open the eyes, the eyes naturally closing, and the like). In addition to motor symptoms as unintended involuntary movement, symptoms of sensory hypersensitivity such as light being dazzling, eye pain, and eye dryness may come to the forefront, and blepharospasm is often misdiagnosed as dry eye. However, unlike dry eye, therapy and treatment used for dry eye, such as instillation, are known to be ineffective. That is, blepharospasm is a disease that exhibits symptoms similar to dry eye but requires a separate factor.

As the current standard therapy for blepharospasm, therapy involving injection of botulinum toxin into the eyelid skin at about 12-16 sites is performed. Both motor symptoms and sensory hypersensitivity symptoms show some improvement, but the current situation is that cases in which sufficiently satisfactory improvement is obtained both subjectively and objectively account for only about 10%. In addition, because the injection site is the eyelid and because the number of injection sites is 10 or more, the procedure is accompanied by pain, and some patients refuse therapy for that reason. The duration of effect is limited to about 2-3 months, and satisfactory therapy results often are not obtained.

In addition, there is also a therapy method in which eyelid-opening assistance and photophobia reduction are performed using crutch glasses, light-shielding glasses, and the like. However, no method by which sufficient therapeutic effects are obtained only by use of external equipment has been found. As surgery, there are also methods using frontalis suspension surgery, levator palpebrae superioris shortening surgery, eyelid skin resection, orbicularis oculi muscle resection, and the like. However, surgery imposes large physical burdens, time burdens, and economic burdens on patients, and no method that can be considered to provide sufficient therapeutic effects in comparison with such burdens has been found.

Due to these circumstances, a new therapeutic method for blepharospasm is strongly desired.

Meanwhile, as a general analgesic and ophthalmic solution for the eye, a technology using tramadol, which is a µ-opioid receptor agonist, has been developed.

Patent Document 1 discloses, in an aqueous composition topically administrable to a human or animal eye, an aqueous composition that includes an aqueous solution of at least one water-soluble polymer ophthalmic lubricating medium and an effective amount of at least one water-soluble analgesic composition, and more specifically, discloses a composition for topical administration to a human or animal eye, the composition including an aqueous solution of at least one water-soluble polymer ophthalmic lubricating medium and an effective amount of tramadol, and being for treating a pathological condition involving a deficiency of natural tears in the human or animal eye.

This technology seeks to provide a topically applicable analgesic preparation that is effective for the eye, for reducing pain caused by therapy or for reducing pain in ocular infections and general pathological conditions, by using tramadol, which is known as an analgesic, for pathological conditions involving a deficiency of natural tears in a human or animal eye (dry eye and the like).

### Citation List

### Patent Document

Patent Document 1: Japanese Unexamined Patent Application, First Publication No. 2017-141243

### SUMMARY OF INVENTION

### Technical Problem

As described above, as treatment methods such as therapeutic methods for blepharospasm, only methods that impose a large burden on patients and cannot necessarily be said to have sufficient therapeutic effects have been found.

The technology of Patent Document 1 is a composition for ophthalmic instillation therapy for dry eye and the like; however, although blepharospasm may be misdiagnosed as dry eye, blepharospasm is a disease completely different from dry eye, and as described above, therapy and treatment for dry eye are ineffective.

In addition, effective treatment is also desired for ocular hypersensitivity such as asthenopia, symptoms of surrounding dystonia, and symptoms of dystonia that have spread from the eyelid.

In addition, in examining therapeutic effects on these symptoms, model animals reproducing symptoms such as blepharospasm, for example, model mice, are also desired.

The present invention has been made in view of the circumstances as described above, and an object of the present invention is to provide a pharmaceutical composition, a blepharospasm treatment agent, and a hypersensitivity or dystonia treatment agent, which have a high effect as treatment such as therapy for blepharospasm and the like, which have conventionally been difficult to treat or for which effective treatment have not been performed, and impose a small burden on patients, and a symptom model mouse for symptoms such as blepharospasm, hypersensitivity, or dystonia.

### Solution to Problem

In order to solve the above problems, the present invention has the following aspects.
[1] A pharmaceutical composition containing tramadol or a salt thereof as an active ingredient, for administration to and treatment of a subject exhibiting symptoms of one of the group consisting of blepharospasm, hypersensitivity, and dystonia in the face or head and neck.
[2] The pharmaceutical composition according to [1], containing 0.01 to 2.0% w/v of the tramadol or a salt thereof.
[3] The pharmaceutical composition according to [1] or [2], which is one of the group consisting of an ophthalmic solution, an injection, an ophthalmic ointment, and a preparation for application to eyelid skin.
[4] The pharmaceutical composition according to any one of [1] to [3], which is an ophthalmic solution and includes artificial tears, a water-soluble polymer ophthalmic lubricating medium, or a surfactant.
[5] A blepharospasm treatment agent, including the pharmaceutical composition according to any one of [1] to [4].
[6] A hypersensitivity or dystonia treatment agent in the face or head and neck, including the pharmaceutical composition according to any one of [1] to [4].
[7] A symptom model mouse of blepharospasm, hypersensitivity, or dystonia, for evaluating the pharmaceutical composition according to any one of [1] to [4] or treatment using the pharmaceutical composition,
   the symptom model mouse being a mouse that has been subjected to stress by a platform or air blowing.
[8] The symptom model mouse according to [7], in which the stress by the platform is applied by placing the mouse on a swingable platform,
   the stress by the air blowing is applied by blowing air toward the front and rear of the mouse on the platform, and
   the mouse has been subjected to the stress by the platform and the air blowing for 4 hours or more per day for 14 days or more.
[9] The symptom model mouse according to [7] or [8], in which the stress by the platform is applied by placing water adjacent to the mouse.

In addition, aspects of the invention of the present application also include the following aspects.
[1A] A composition for medical use containing tramadol or a salt thereof as an active ingredient, for administration to and treatment of a subject exhibiting symptoms of blepharospasm or hypersensitivity or dystonia in the face or head and neck.
[2A] The composition for medical use according to [1A], containing 0.01 to 2.0% w/v of the tramadol or a salt thereof.
[3A] A medicament including the composition for medical use according to [1A] or [2A].
[4A] The medicament according to [3A], which is one of the group consisting of an ophthalmic solution, an injection, an ophthalmic ointment, and a preparation for application to eyelid skin.
[5A] The ophthalmic solution according to [3A], including artificial tears, a water-soluble polymer ophthalmic lubricating medium, or a surfactant.
[6A] A blepharospasm treatment agent, including the composition for medical use according to [1A] or [2A].
[7A] A hypersensitivity or dystonia treatment agent in the face or head and neck, including the composition for medical use according to [1A] or [2A].

### Advantageous Effects of Invention

According to the present invention, it is possible to provide a pharmaceutical composition, a medicament, a blepharospasm treatment agent, and a hypersensitivity or dystonia treatment agent, which have a high effect as treatment such as therapy for blepharospasm and the like, which have conventionally been difficult to treat or for which effective treatment has not been performed, and impose a small burden on patients, and a symptom model mouse for symptoms such as blepharospasm, hypersensitivity, or dystonia.

### BRIEF DESCRIPTION OF DRAWINGS

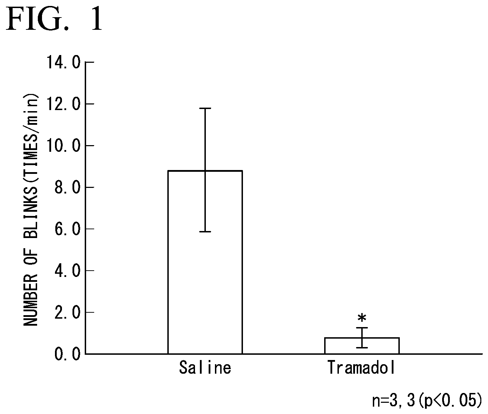

[FIG. 1] A graph showing the results of the number of blinks when eye drops were administered to symptom model mice.

### DESCRIPTION OF EMBODIMENTS

Hereinafter, embodiments of the pharmaceutical composition, blepharospasm treatment agent, and hypersensitivity or dystonia treatment agent according to the present invention will be described. However, the present invention is not limited to the following embodiments.

### (Pharmaceutical Composition)

The pharmaceutical composition according to the present embodiment is a pharmaceutical composition containing tramadol or a salt thereof as an active ingredient, for administration to and treatment of a subject exhibiting symptoms of blepharospasm or hypersensitivity or dystonia in the face or head and neck. The pharmaceutical composition broadly includes not only so-called medicaments but also compositions used for medical purposes (compositions for medical use).

Tramadol (C₁₆H₂₅NO₂, CAS No.: 27203-92-5) is known as one of opioid analgesics, and has been reported to have an action as a partial agonist of the µ-opioid receptor and a serotonin/noradrenaline reuptake inhibitory action.

The salt of tramadol can be appropriately selected as long as the salt is a pharmaceutically acceptable salt. In addition, various derivatives of tramadol can be selected as long as the derivatives are within pharmaceutically and physiologically acceptable ranges and do not reduce pharmacological effects.

The pharmaceutical composition of the present embodiment is a pharmaceutical composition for administration to and treatment of a subject exhibiting symptoms such as blepharospasm.

Blepharospasm is a disease accompanied by unintended involuntary movement (dystonia) of the eyelid (dystonia in the eye or eyelid includes symptoms such as the eyes not opening despite wanting to open the eyes and the eyes naturally closing), and specifically, the involuntary movement exhibits symptoms such as difficulty opening the eyes and feeling more comfortable with the eyes closed. Blepharospasm also exhibits symptoms such as light being dazzling and eye pain.

As a result of such symptoms, blepharospasm may exhibit symptoms similar to dry eye and the like, for example, symptoms such as eye dryness and pain. For this reason, blepharospasm may be misdiagnosed as dry eye or another eye disease exhibiting these symptoms.

Blepharospasm in the present embodiment refers to a disease accompanied by unintended involuntary movement of the eyelid. That is, blepharospasm in the present embodiment does not refer to symptoms of dry eye only, symptoms only of a pathological condition involving a deficiency of natural tears, for example, symptoms accompanied only by eye dryness or pain and not accompanied by involuntary movement.

Regarding the pharmaceutical composition of the present embodiment, the target disease further includes symptoms of ocular hypersensitivity. The pharmaceutical composition of the present embodiment is also effective for symptoms of head and neck dystonia or hypersensitivity around the eyes. In addition, the pharmaceutical composition is expected to be effective also in cases where symptoms have spread from the eyelid to dystonia and the like of the face to the head and neck.

Examples of these symptoms include asthenopia. More specific examples include asthenopia associated with near work such as reading.

In the present specification, blepharospasm and other diseases targeted by the present embodiment, such as hypersensitivity or dystonia in the face or head and neck, may be collectively referred to as "blepharospasm and the like".

A subject exhibiting symptoms of blepharospasm, hypersensitivity, or dystonia targeted by the present embodiment can be appropriately selected from humans and other animals. Examples of other animals mainly include mammals. Specific examples of mammals include livestock animals (pigs, sheep, goats, cattle, horses, and the like), companion animals (dogs, cats, and the like), experimental animals (mice, guinea pigs, hamsters, rats, other murines, rabbits, and the like), and other animals (marmosets, chimpanzees, and other monkeys). In the present specification, when the term "patient" is used, the term mainly refers to a human subject, but is not limited thereto.

Purposes of treatment for administering the pharmaceutical composition of the present embodiment to and treating a patient with blepharospasm and the like broadly include purposes of medical treatment such as therapy, prevention, and prevention of recurrence after therapy.

As described later, examples of the treatment include instillation and the like, but the treatment is not limited thereto.

The pharmaceutical composition of the present embodiment preferably contains 0.01 to 2.0% w/v of the tramadol or a salt thereof.

In addition, the pharmaceutical composition more preferably contains 0.5 to 1.5% w/v of the tramadol or a salt thereof, and still more preferably contains 0.8 to 1.2% w/v of the tramadol or a salt thereof.

The dose, administration interval, administration method, and administration route are not particularly limited and can be appropriately selected according to conditions of the subject to be treated, for example, age, body weight, symptoms, target site, and the like.

The pharmaceutical composition may be in the form of a medicament including the components. The medicament is preferably in a form for administration to the eye and the area around the eye for use in treatment.

Specifically, the medicament may be an ophthalmic solution, an injection, an ophthalmic ointment, or a preparation for application to eyelid skin.

### (Ophthalmic Solution)

The pharmaceutical composition of the present embodiment may be an ophthalmic solution. The pharmaceutical composition is preferably a solution in a form suitable for instillation, and more preferably an aqueous solution. The concentration can be appropriately selected according to the concentration of the pharmaceutical composition.

The ophthalmic solution of the present embodiment may include, in addition to the pharmaceutical composition, any pharmaceutically acceptable optional component within a range that does not impair the effects provided by the formulation of the present embodiment. Examples of the optional component include a thickener, a preservative, a tonicity agent, a dispersant (such as polysaccharides), a buffer, a pH adjuster, and the like.

The thickener adjusts the viscosity (form) of the drug. Examples of the thickener include cellulose-based thickeners such as methylcellulose, hydroxyethylcellulose, and hydroxypropylmethylcellulose; vinyl-based thickeners such as carboxyvinyl polymer, polyvinyl alcohol (complete or partial saponified product), and polyvinylpyrrolidone; sodium alginate, sodium chondroitin sulfate, macrogol, and the like.

The preservative improves the storage stability of the drug. Examples of the preservative include invert soaps such as benzalkonium chloride, benzethonium chloride, and chlorhexidine gluconate; parabens such as methylparaben, ethylparaben, propylparaben, and butylparaben; and alcohols such as chlorobutanol, phenylethyl alcohol, and benzyl alcohol.

In a drug such as an eye drop, the tonicity agent suggests irritation to the eye. Examples of the tonicity agent include sugars such as glucose; polyhydric alcohols such as propylene glycol, glycerin, mannitol, sorbitol, and xylitol; inorganic salts such as sodium chloride and potassium chloride; and the like.

The dispersant suppresses aggregation of components contained in the drug. Examples of the dispersant include α-cyclodextrin, β-cyclodextrin, γ-cyclodextrin, and derivatives thereof.

The pH adjuster adjusts the pH of the drug. Examples of the pH adjuster include dilute hydrochloric acid, sodium hydroxide, and the like.

The buffer stabilizes the pH of the drug. Examples of the buffer include sodium citrate hydrate, sodium acetate hydrate, sodium hydrogen carbonate, trometamol, boric acid, borax, sodium hydrogen phosphate hydrate, sodium dihydrogen phosphate, and the like.

The ophthalmic solution of the present embodiment may include artificial tears, a water-soluble polymer ophthalmic lubricating medium, or a surfactant.

Examples of the artificial tears include conventionally known components that assist good lubrication of the eye, and the artificial tears are also referred to as an ophthalmic lubricant composition. Examples of the artificial tears include a lubricating preparation containing sodium hyaluronate, hypromellose, or carbomer gel.

A water-soluble polymer ophthalmic lubricating medium that includes a water-soluble polymer as a component that assists lubrication of the eye can be used. Examples of these water-soluble polymers include polyvinyl alcohol (PVA), polyethylene glycol (PEG), carbomer, polyvinylpyrrolidone (PVP), and the like.

A surfactant can lower the surface tension of a tear fluid composition and can therefore improve wetting of the surface of the eye. The surfactant may also include a nonionic surfactant and an ionic surfactant. As the nonionic surfactant, polysorbate, poloxamer, a tetrafunctional block copolymer, and the like can be used.

These components can be used within a pharmaceutically acceptable range.

In addition, the ophthalmic solution of the present embodiment may be free of a preservative, and further may be free of other components of a solvent (water) component and tramadol or a salt thereof as an active ingredient. Tramadol or a salt thereof is stable in a solvent over a long period of time and therefore can be used as an ophthalmic solution without containing other components.

### (Other Medicaments)

The pharmaceutical composition may be an injection or an ophthalmic ointment. The preparations may include components similar to those of the ophthalmic solution.

The pharmaceutical composition may be a preparation for application to eyelid skin. In addition to the above, the preparations may include components similar to those of a general skin ointment. The preparations for application may be in forms such as a liquid preparation for application, a lotion, a milky lotion, a spray, a cream, a gel, or the like. In addition, the preparations may be in a form included in other external preparations such as a skin lotion and a beauty serum.

### (Blepharospasm Treatment Agent, and Hypersensitivity or Dystonia Treatment Agent)

The therapeutic agent treatment agent for blepharospasm of the present embodiment, and the hypersensitivity or dystonia treatment agent, include the pharmaceutical composition.

The treatment agent for blepharospasm and the like of the present embodiment can take a conventionally known form. The treatment agent for blepharospasm and the like of the present embodiment preferably takes the form of an ophthalmic solution. When the treatment agent for blepharospasm and the like of the present embodiment takes the form of an ophthalmic solution, the treatment agent may have a formulation (composition and the like) similar to that of the ophthalmic solution. As the form of the preparation, forms conventionally adopted as preparation forms for each administration form can be employed without particular limitation.

The administration conditions of the treatment agent for blepharospasm and the like of the present embodiment may be appropriately selected according to the form of the therapeutic agent and the symptoms, body weight, age, sex, and the like of the patient.

For example, when the therapeutic agent takes the form of the ophthalmic solution described above, the administration conditions may be selected from conventionally known administration conditions of ophthalmic solutions. For example, administration may be performed once to six times per day, preferably two to four times per day, and administration may be continued for 1 week or more, preferably 1 month or more.

The treatment agent for blepharospasm and the like of the present embodiment can be widely used for the above-described purposes, for treatment for administration to and treatment of a subject exhibiting symptoms of blepharospasm and the like.

### (Symptom Model Mouse)

The symptom model mouse of the present embodiment is a symptom model mouse for evaluating a pharmaceutical composition or treatment using the pharmaceutical composition.

That is, the symptom model mouse of the present embodiment is a model mouse of symptoms of a subject exhibiting the above-described symptoms, symptoms of blepharospasm, or symptoms of hypersensitivity or dystonia in the face or head and neck, and is used for verification of the symptoms and for evaluating effects of treatment for the symptoms, particularly treatment using a pharmaceutical composition.

Examples of the pharmaceutical composition include the above-described pharmaceutical composition containing tramadol or a salt thereof as an active ingredient.

The symptom model mouse of the present embodiment is a symptom model mouse that has been subjected to stress by a platform or air blowing.

That is, the symptom model mouse of the present embodiment is caused to exhibit the above-described symptoms of blepharospasm or hypersensitivity or dystonia in the face or head and neck by applying a certain stress to the mouse for a certain period of time.

The stress by the platform can be applied by providing an unstable platform. For example, the stress can be applied by placing the mouse on a swingable platform. Examples of the swingable platform include a platform suspended by unfixed means, for example, a string or the like. In addition, the platform itself is also preferably one whose position is difficult to maintain, and, for example, a rod or a long-sized platform having a size in the width direction that is equal to or smaller than the width of the body of the mouse can be used.

The stress by the platform may apply mental stress (psychological stress) in addition to the instability of the platform. For example, the platform may be provided at a high place.

For example, the stress by the platform may be applied by placing water adjacent to the mouse.

Placing water adjacent to the mouse refers to placing water near the mouse such that the mouse easily comes into contact with the water. For example, the water is preferably placed near the unstable platform. In addition, for example, placing water at a position lower than the swingable platform is also more preferable.

Specific examples of such a platform may include a platform in which a rod-shaped platform is suspended by a string above a water tank containing water in a swingable state. Because the water is placed such that the mouse can easily fall from the platform and come into contact with the water, strong mental stress can be applied to the mouse, and one risk factor for blepharospasm can be strongly applied.

Here, water broadly refers to aqueous solutions and the like in which water accounts for a majority of the mass among components, and purity and the like are not limited.

The stress by the air blowing is stress applied to the eyes of the mouse, and also has an effect of increasing the stress from the instability of the platform. The air blowing causes eye dryness and prolonged staring (suppression of blinking), and can strongly provide one risk factor for blepharospasm.

The stress by the air blowing is applied by blowing air toward the front or rear of the mouse on the platform. The means, strength, and the like of the air blowing may be appropriately selected according to conditions under which the various symptoms appear. In the present embodiment, air is blown from both the front and the rear toward the mouse on the platform using a blower.

The conditions in terms of time and number of times for applying the stress may be appropriately selected according to conditions under which the various symptoms appear; however, as a guide, by applying the stress by the platform and the stress by the platform and air blowing for 4 hours or more per day for 14 days or more, the above-described symptoms of blepharospasm or hypersensitivity or dystonia in the face or head and neck can be confirmed. In particular, symptoms of blepharospasm can be confirmed by applying stress for 4 hours per day for 18 days or more.

In addition, conditions such as other stresses corresponding to risk factors for blepharospasm may be applied to the model mouse. Sex hormone changes, prolonged suppression of blinking, and mental stress are expected as three risk factors for blepharospasm.

For example, the symptom model mouse may be produced by applying the stress using an aged mouse. Aged mice are considered to be greatly affected by sex hormone changes. In addition, as sex hormone changes, mice that have undergone excision or functional impairment of organs related to hormone secretion, such as ovaries, may be used.

In order to evaluate a pharmaceutical composition or treatment using the pharmaceutical composition by the symptom model mouse of the present embodiment, the evaluation can be performed by using a control mouse not subjected to the stress and a mouse not subjected to the pharmaceutical composition or treatment using the pharmaceutical composition, and making a comparison with a mouse subjected to the treatment.

### (Effects of Present Embodiment)

According to the present embodiment, it is possible to provide a pharmaceutical composition, a blepharospasm treatment agent, a hypersensitivity or dystonia treatment agent, which have a high effect as treatment such as therapy for blepharospasm and the like, which have conventionally been difficult to treat or for which effective treatment has not been performed, and impose a small burden on patients, and a symptom model mouse.

The present inventors have found that symptoms of blepharospasm are improved by using tramadol hydrochloride, which is a µ-opioid receptor agonist, as an ophthalmic instillation. Because a µ-opioid receptor agonist is expected to have an analgesic action, the possibility that the µ-opioid receptor agonist improves sensory hypersensitivity symptoms such as pain and dryness in blepharospasm patients may be imagined; however, when ophthalmic instillation of tramadol hydrochloride was actually performed in blepharospasm patients, motor symptoms of the eyelid were improved independently of improvement in sensory hypersensitivity symptoms.

In addition, the composition of the present embodiment also exerted effects on symptoms of ocular hypersensitivity such as asthenopia. Similarly, the composition is considered to exert effects also on similar symptoms in other sites to which blepharospasm spreads, for example, the face, head and neck, and upper body.

Conventionally, as therapeutic effects of tramadol, analgesic effects have been widely known, and certain effects on dry eye have also been known. However, improvement effects on other sensory hypersensitivity and motor symptoms have not been known.

The present embodiment, unlike a simple analgesic effect, exhibited an improvement effect on motor symptoms, and topical administration of tramadol hydrochloride to the eye is capable of improving motor symptoms in blepharospasm patients.

**In** addition, according to the present embodiment, a symptom model mouse reproducing symptoms corresponding to symptoms of blepharospasm or ocular hypersensitivity such as asthenopia can be obtained, and the symptom model mouse can be used for verification of these diseases, symptoms, and therapeutic methods.

### (Another Aspect of Present Embodiment)

The present embodiment, as another aspect, also includes the following aspects.

Another aspect of the present embodiment is a method for manufacturing a blepharospasm treatment agent using the pharmaceutical composition.

Another aspect of the present embodiment is use of the pharmaceutical composition for manufacturing a blepharospasm treatment agent.

Another aspect of the present embodiment is use of the pharmaceutical composition for treatment of blepharospasm and the like.

Another aspect of the present embodiment is use of the pharmaceutical composition for use in treatment of blepharospasm and the like.

Another aspect of the present embodiment is a method for treating blepharospasm and the like using the pharmaceutical composition.

The embodiments of the present invention have been described above, but the present invention is not limited to the above embodiments, and various modifications can be made.

### Examples

Hereinafter, the effects of the present invention will be made clearer by Examples and Comparative Examples. The present invention is not limited only to the following Examples, and can be implemented with appropriate modifications within a range that does not change the gist of the present invention.

### (Test Example 1)

A 5-fold diluted solution was adjusted from a commercially approved tramadol injection solution (Tramadol Hydrochloride OD Tablets 25 mg "KO", Kotobuki Pharmaceutical Co., Ltd.), the diluted solution was subjected to suction filtration sterilization, dispensed and filled into ophthalmic solution bottles, and stored in a refrigerator. As quality testing, samples for quality inspection were extracted, tests were performed for drug assay, properties, pH, osmotic pressure ratio, sterility, insoluble foreign matter, and fine particles, and the pharmaceutical composition was transported and stored in the form of an eye drop as a pharmaceutical composition containing 1% tramadol, and was used in the following administration tests.

Ophthalmic instillation with the eye drops containing the 1% tramadol was administered to nine blepharospasm patients four times per day for one month. Subjective symptoms before and after administration were evaluated using the visual analog scale (VAS) and multiple questionnaires.

The results of administration were as follows:
five of nine cases: both sensory hypersensitivity and motor symptoms improved
two of nine cases: only sensory hypersensitivity improved
one of nine cases: only motor symptoms improved
one of nine cases: neither sensory hypersensitivity nor motor symptoms improved.

The averages for the nine cases were as follows:
sensory hypersensitivity VAS
   before treatment: 56.9 ± 18.9 after treatment: 36.9 ± 21.4 (p = 0.003)
motor symptoms total score
   before treatment: 3.4 ± 1.4 after treatment: 1.8 ± 1.2 (p = 0.02).

Among the nine cases of blepharospasm patients, improvement was observed in both motor symptoms and sensory hypersensitivity symptoms in five cases. Improvement was observed only in sensory hypersensitivity symptoms in two cases and only in motor symptoms in one case. There was one case in which improvement was not observed in either motor symptoms or sensory hypersensitivity symptoms.

That is, by performing ophthalmic instillation with 1% tramadol, both motor symptoms and sensory hypersensitivity symptoms were improved in more than half of the blepharospasm patients. **In** addition, results were obtained indicating that, in most patients, improvement was observed in either motor symptoms or sensory hypersensitivity symptoms.

In the conventional technology, no technology had been found that serves as a therapy means capable of providing a high improvement effect such as reliable subjective symptoms for symptoms of blepharospasm and providing improvement effects in various patients with a high probability, but use of the pharmaceutical composition of the present embodiment in therapy as an ophthalmic solution or therapeutic agent is considered to provide effective treatment of blepharospasm.

### (Test Example 2)

A symptom model mouse reproducing symptoms corresponding to blepharospasm was produced, the eye drops containing the tramadol were administered to the symptom model mouse, and the effect of the pharmaceutical composition due to alleviation of symptoms was verified.

When stress (risk factors) by the platform and air blowing overlap in mice, it has been confirmed by a preliminary separate test that a blepharospasm-like phenotype appears compared with mice not subjected to these stresses (not shown). In addition, when the number of blinks was compared after ophthalmic instillation once per day for 3 days between a tramadol ophthalmic instillation administration group and a physiological saline ophthalmic instillation group, with n=4 eyes each, in mice not subjected to stress, no significant difference in the number of blinks was observed between the two groups, and no other clear difference was observed in the eyes or the whole body, and it has also been confirmed that tramadol has no other effects on mice without symptoms corresponding to blepharospasm (not shown).

Six C57BL/6JJcl, 38-week-old female mice were subjected to stress by a platform and air blowing, thereby producing symptom model mice in which symptoms corresponding to blepharospasm appeared.

An unstable suspension bridge platform made of a rod-shaped member suspended by a string was installed above a cage filled with water. The stress by the platform and air blowing was applied by placing the mice on the platform and blowing air from the front and rear directions using blowers (fans).

The stress was applied for 4 hours per day (excluding Saturdays and Sundays), and was continued for approximately 3 weeks.

In parallel with the stress environment, the eye drops containing tramadol similar to those of Test Example 1 were administered to three of these symptom model mice. The group of the other three mice was treated with physiological saline only. On day 18, administration of tramadol was stopped, and on day 21, the number of blinks was measured.

The number of blinks was measured by restraining awake mice and measuring the number of blinks for 1 minute.

FIG. 1 is a graph showing the results of the number of blinks when eye drops were administered to symptom model mice. In the figure, Saline indicates mice treated with physiological saline only, and Tramadol indicates mice administered the eye drops. Administration of the eye drops significantly decreased the number of blinks.

The above results showed that, in model mice for symptoms of blepharospasm, the symptoms were alleviated by administration of the eye drops containing tramadol.

The embodiments of the present invention have been described above, but these embodiments are presented as examples and are not intended to limit the scope of the invention. These embodiments can be implemented in various other forms, and various omissions, replacements, and changes can be made without departing from the gist of the invention. These embodiments and modifications thereof are included in the scope and gist of the invention, and are also included in the invention described in the claims and the scope of equivalents thereof.

### INDUSTRIAL APPLICABILITY

According to the present invention, it is possible to provide a pharmaceutical composition, a blepharospasm treatment agent, a hypersensitivity or dystonia treatment agent, which have a high effect as treatment such as therapy for blepharospasm and the like, which have conventionally been difficult to treat or for which effective treatment has not been performed, and impose a small burden on patients, and a symptom model mouse.

## Claims

1. A pharmaceutical composition comprising tramadol or a salt thereof as an active ingredient, for administration to and treatment of a subject exhibiting symptoms of one of the group consisting of blepharospasm, hypersensitivity, and dystonia in a face or head and neck.

2. The pharmaceutical composition according to claim 1, comprising 0.01 to 2.0% w/v of the tramadol or a salt thereof.

3. The pharmaceutical composition according to claim 1 or 2, which is one of the group consisting of an ophthalmic solution, an injection, an ophthalmic ointment, and a preparation for application to eyelid skin.

4. The pharmaceutical composition according to claim 3, which is an ophthalmic solution and comprises artificial tears, a water-soluble polymer ophthalmic lubricating medium, or a surfactant.

5. A blepharospasm treatment agent, comprising the pharmaceutical composition according to claim 1 or 2.

6. A hypersensitivity or dystonia treatment agent in the face or head and neck, comprising the pharmaceutical composition according to claim 1 or 2.

7. A symptom model mouse of blepharospasm, hypersensitivity, or dystonia, for evaluating the pharmaceutical composition of claim 1 or treatment using the pharmaceutical composition,
the symptom model mouse being a mouse that has been subjected to stress by a platform or air blowing.

8. The symptom model mouse according to claim 7, wherein
the stress by the platform is applied by placing the mouse on a swingable platform,
the stress by the air blowing is applied by blowing air toward the front and rear of the mouse on the platform, and
the mouse has been subjected to the stress by the platform and the air blowing for 4 hours or more per day for 14 days or more.

9. The symptom model mouse according to claim 7 or 8, wherein the stress by the platform is applied by placing water adjacent to the mouse.
